# EUROPEAN PATENT APPLICATION

(11) **EP 4 566 600 A1**
(43) Date of publication of application: **11.06.2025**
(21) Application number: 23849798.6
(22) Date of filing: 28.06.2023
(51) Int. Cl.: A61K 31/353, A23L 33/10, A61K 31/7048, A61P 25/18, A61P 43/00

(54) **AFFECTIVE DISORDER AMELIORATING AGENT**

(30) Priority: 02.08.2022 JP 2022123541
(71) Applicant: National University Corporation Kobe University, Kobe-shi, Hyogo 657-8501 (JP)
(72) Inventor: SHIRAI, Yasuhito, Kobe-shi, Hyogo 657-8501 (JP)
(74) Representative: Müller-Boré & Partner Patentanwälte PartG mbB
(86) International application number: PCT/JP2023/023966
(87) International publication number: WO 2024/029239

(57) **Abstract**

The present invention provides a new affective disorder ameliorating agent. A PKCγ activity inhibitor is useful as an active ingredient for an affective disorder ameliorating agent.

## Description

### Technical Field

The present invention relates to an affective disorder ameliorating agent. More specifically, the present invention relates to an affective disorder ameliorating agent effective for mania.

### Background Art

Among mental disorders, a state indicating an affective disorder indicating excessive depression caused by sadness over a long period of time (depression), excessive elation caused by joy (mania), or both of them is referred to as a mood disorder (also referred to as an affective disorder) (NPL 1).

Among the mood disorders, examples of the disease state including "mania" include bipolar disorder and mania (manic psychosis). In bipolar disorder, a depressed state and a manic state or a hypomanic state (mild manic state) are alternately observed. In manic psychosis, a manic state appears extremely. The manic state is characterized by excessive physical activity and elation that is significantly disproportionate to the situation in which the person is placed (NPL 2).

Bipolar disorder can have a significant impact on social life. In particular, in the "manic" state, there is a case in which a serious situation occurs due to an erratic behavior, which is particularly serious (NPL 3). In bipolar disorder, the risk of suicide is particularly high during a mixed episode in which a depressed state and a manic state or a hypomanic state occur at a time (NPL 2).

In addition, it has been reported that, in mania, there is a possibility of making a suicide attempt extremely carelessly due to a small matter as a trigger, and there is a disease image in which there is a high risk of suicide attempt in mania, and there is a possibility of suicide attempt also in a hypomanic period, from the senseless reality judgment and self-control attentive ability (NPL 4).

The recent COVID-19 pandemic is said to have disrupted people's daily lives and adversely affected their mental health. The UN has warned of a COVID-19 related mental health crisis, and in fact it has been reported that intense concerns about both health and economic consequences in the COVID-19 pandemic were associated with an increased likelihood of having mental disorders (NPL 5).

As drugs acting in mania, lithium (trade name: Limas and the like), valproic acid (trade name: Depakene and the like), carbamazepine (trade name: Tegretol and the like), and the like are known. These drugs must be applied carefully considering the risk of side effects.

On the other hand, attempts have also been made to ingest a component having a relaxation effect from food. For example, the striatum of the brain and the like are said to be involved in emotions, and it has been reported that when theanine, a green tea component, is injected into the brain striatum of a living rat, the release amount of dopamine increases (NPL 6), and that the electroencephalographic measurement after theanine ingestion in humans signified relaxation without causing drowsiness (NPL 7).

### Citation List

### Non Patent Literature

NPL 1: William Coryell, MSD Manual Consumer Version 10. MENTAL HEALTH DISORDERS "Overview of Mood Disorders", [online], May 2018, Merck & Co., Inc., Rahway, NJ, USA, [searched on July 5, 2022], Internet <URL: https://www.msdmanuals.com/ja-jp/%E3%83%9B%E3%83%BC%E3%83%A0/10-%E5%BF%83%E3%81%AE%E5%8 1%A5%E5%BA%B7%E5%95%8F%E9%A1%8C/%E6%B0%97%E5%88%86%E9%9 A%9C%E5%AE%B3/%E6%B0%97%E5%88%86%E9%9A%9C%E5%AE%B3%E3% 81%AE%E6%A6%82%E8%A6%81>
NPL 2: William Coryell, MSD Manual Consumer Version 10. MENTAL HEALTH DISORDERS "Bipolar Disorder (Manic-Depressive Disorder)", [online], May 2018, Merck & Co., Inc., Rahway, NJ, USA, [searched on July 5, 2022], Internet <URL: https://www.msdmanuals.com/ja-jp/%E3%83%9B%E3%83%BC%E3%83%A0/10-%E5%BF%83%E3%81%AE%E5%8 1%A5%E5%BA%B7%E5%95%8F%E9%A1%8C/%E6%B0%97%E5%88%86%E9%9 A%9C%E5%AE%B3/%E5%8F%8C%E6%A5%B5%E6%80%A7%E9%9A%9C%E5 %AE%B3#v748316#ja>
NPL 3: Supervised by Kaoru Sakamoto, Professor, Department of Psychiatry, Tokyo Women's Medical University, NHK Heart-net, Welfare information site, Information room for young's mental illness "Bipolar Disorder", [online], May 2018, Japan Broadcasting Corporation, [searched on July 5, 2022], Internet <URL: https://www.nhk.or.jp/heart-net/kokoro/ss/index04.html>
NPL 4: Suicide attempt in mania (part 2) Tokyo Women's Medical University, Vol. 58, No. 12, p. 1237-1258 (December 1988)
NPL 5: P. Winkler, T. Formanek, K. Mlada, A. Kagstrom, Z. Mohrova, P. Mohr and L. Csemy, Epidemiology and Psychiatric Sciences 29, e173, 1-8.
NPL 6: H. Yokogoshi, M. Kobayashi, M. Mochizuki & T. Terashima: Neurochem. Res., 23, 667 (1998)
NPL 7: Lekh Raj Juneja, Djong -Chi Chu, Tsutomu Okubo, Yukiko Nagato, Hidehiko Yokogoshi, Trends Food Sci. Technol., 10, 199 (1999).

### Summary of Invention

### Technical Problem

Considering the side effects of drugs currently applied to affective disorders, it is significant to expand the scope of selection to other active ingredients. Among them, there is a merit that the ingredient that calms the emotion can be ingested in food. However, the point of action of such a food component is unclear, and the effect is far from the effect brought about by the drug. Therefore, it is desirable to provide a new component that responds to affective disorder based on a clear mechanism of action.

Therefore, an object of the present invention is to provide a new affective disorder ameliorating agent with a clear mechanism of action.

### Solution to Problem

The present inventor has confirmed that, using a DGKβ knockout mouse that has been found to exhibit enhanced PKCγ phosphorylation (abnormal PKCγ activity) in the brain as a unique affective disorder model, the affective disorder is ameliorated through reduced PKCγ phosphorylation (normalization of PKCγ activity) by allowing this affective disorder model to take lithium, and then found that the affective disorder is ameliorated together with the normalization of PKCγ activity in the brain by allowing this affective disorder model to take a PKCγ activity inhibitor. The present invention has been accomplished by further studies based on this knowledge.

That is, the present invention provides inventions of the following aspects.

Item 1. An affective disorder ameliorating agent containing a PKCγ activity inhibitor as an active ingredient.

Item 2. The affective disorder ameliorating agent according to item 1, in which the PKCγ activity inhibitor is selected from the group consisting of scutellarin, baicalin, baicalein, luteolin, apigenin, apigenin glucoside, and salts thereof.

Item 3. The affective disorder ameliorating agent according to item 1, in which the affective disorder is a disease state or condition including mania.

Item 4. A food containing the affective disorder ameliorating agent according to item 1.

Item 5. A PKCγ activity inhibitor containing, as an active ingredient, a compound selected from the group consisting of baicalin, baicalein, luteolin, apigenin, apigenin glucoside, and salts thereof.

Item 6. A method for ameliorating affective disorder, including administering an effective amount of a PKCγ activity inhibitor to a subject in need of affective disorder amelioration.

Item 7. A method for ameliorating a disease state or condition caused by abnormal PKCγ activity, including administering an effective amount of a compound selected from the group consisting of baicalin, baicalein, luteolin, apigenin, apigenin glucoside, and salts thereof to a subject in need of amelioration of a disease state or condition caused by abnormal PKCγ activity.

Item 8. Use of a PKCγ activity inhibitor for the production of a composition for ameliorating affective disorder.

Item 9. Use of a compound selected from the group consisting of baicalin, baicalein, luteolin, apigenin, apigenin glucoside, and salts thereof for the production of a PKCγ activity inhibitor.

Item 10. A PKCγ activity inhibitor for use in ameliorating affective disorder.

Item 11. A compound selected from the group consisting of baicalin, baicalein, luteolin, apigenin, apigenin glucoside, and salts thereof, for use in ameliorating a disease state caused by abnormal PKCγ activity.

Item 12. Non-pharmaceutical use of a PKCγ activity inhibitor for amelioration of affective disorder.

Item 13. Non-pharmaceutical use of a compound selected from the group consisting of baicalin, baicalein, luteolin, apigenin, apigenin glucoside, and salts thereof for ameliorating a condition caused by abnormal PKCγ activity.

### Advantageous Effects of Invention

According to the present invention, a new affective disorder ameliorating agent with a clear mechanism of action is provided.

### Brief Description of Drawings

[Fig. 1] Fig. 1 shows the results of confirming the activity of PKCs in the cerebral cortex of mice administered with lithium by Western blotting.
[Fig. 2] Fig. 2 shows the results of confirming the effect of ameliorating affective disorder in mice orally administered with a PKCγ activity inhibitor by light/dark box test.
[Fig. 3] Fig. 3 shows the results of confirming the activity of PKCs in the cerebral cortex of mice administered with a PKCγ activity inhibitor by Western blotting.
[Fig. 4] Fig. 4 shows the results of confirming the activity of PKCs in the hippocampus of mice administered with a PKCγ activity inhibitor by Western blotting.
[Fig. 5] Fig. 5 shows the results of confirming the PKCγ activity inhibitory action of various flavonoids.

### Description of Embodiments

### 1. Affective disorder ameliorating agent

The affective disorder ameliorating agent of the present invention contains a PKCγ activity inhibitor as an active ingredient.

### Active ingredient

The PKCγ activity inhibitor is not particularly limited as long as it is a compound having an action of inhibiting phosphorylation of PKCγ. Preferable examples of the PKCγ activity inhibitor used in the present invention include flavonoids such as scutellarin (the following formula (1)), baicalin (the following formula (2)), baicalein (the following formula (3)), luteolin (the following formula (4)), apigenin (the following formula (5)), apigenin glucoside (the following formula (6)), and salts thereof.

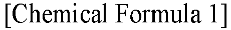

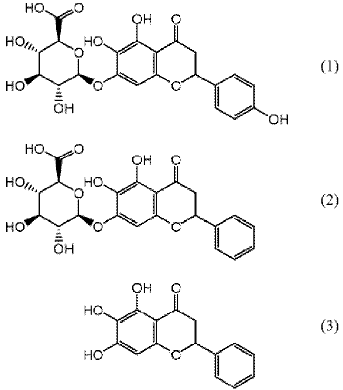

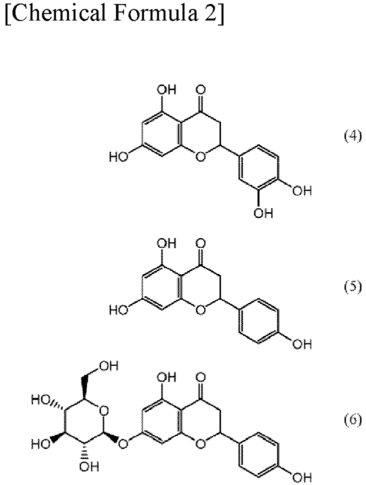

In addition, the salt is not particularly limited as long as it is a pharmaceutically or food acceptable salt, and examples thereof include alkali metal salts such as sodium salts, and the like.

These PKCγ activity inhibitors may be used alone or in combination of two or more. Among these PKCγ activity inhibitors, scutellarin and baicalin are preferred, and scutellarin is more preferred.

The blending amount of the PKCγ activity inhibitor contained in the affective disorder ameliorating agent of the present invention is not particularly limited, and can be appropriately set according to the preparation form, specific use, and the like of the affective disorder ameliorating agent, and is, for example, 40 to 100 wt%, preferably 60 to 90 wt%.

### Other components

The affective disorder ameliorating agent of the present invention may or may not further contain additives and/or bases according to the preparation form and use as long as the effects of the present invention are not impaired, in addition to the above active ingredients. Such additives and bases that may or may not be contained are not particularly limited as long as they are pharmaceutically acceptable, and examples thereof include excipients, binders, disintegrating agents, lubricants, isotonizing agents, plasticizers, dispersants, emulsifiers, solubilizing agents, wetting agents, stabilizers, suspending agents, pressure sensitive adhesives, gelling agents, coating agents, gloss agents, water, fats and oils, waxes, hydrocarbons, fatty acids, higher alcohols, esters, water-soluble polymers, surfactants, metal soaps, lower alcohols, polyhydric alcohols, pH adjusters, buffering agents, antioxidants, ultraviolet inhibitors, preservatives, flavoring agents, fragrances, powders, thickeners, colorants, chelating agents, sweeteners, and the like. These additives may be used alone or in combination of two or more types. Also, the contents of these additives and bases are appropriately set according to the types of additives and bases to be used, the preparation form and use of the affective disorder ameliorating agent of the present invention, and the like.

Moreover, the affective disorder ameliorating agent of the present invention may or may not further contain other nutritional components or pharmacological components as long as the effects of the present invention are not impaired, in addition to the above active ingredients. Such nutritional component or pharmacological component that may or may not be contained is not particularly limited as long as it is pharmaceutically acceptable, and examples thereof include antacids, stomachics, digestive agents, antiflatulents, antispasmodics, mucosal repair agents, antiinflammatory agents, astringents, antiemetics, antitussives, expectorants, antiinflammatory enzymes, sedative-hypnotics, antihistamines, caffeines, cardiotonic diuretics, antibacterial agents, vasoconstrictors, vasodilators, local anesthetics, herbal extracts, vitamins, menthols, and the like. These nutritional components and pharmacological components may be used alone or in combination of two or more types. In addition, the content of these components is appropriately set according to the type of components to be used.

### Preparation form

The form and property of the affective disorder ameliorating agent of the present invention are not particularly limited as long as it contains the above active ingredients.

The administration form of the affective disorder ameliorating agent of the present invention includes both an oral administration form and a parenteral administration form. Therefore, the affective disorder ameliorating agent of the present invention can be prepared as an oral preparation, an injection, a drip, a nasal drop, or the like. Among them, the affective disorder ameliorating agent of the present invention is preferably an oral agent that can be easily administered (ingested) on a daily and/or continuous basis.

Also, the property of the affective disorder ameliorating agent of the present invention may be liquid or solid. Examples of the liquid agent include liquid agents, beverage preparations, emulsions, suspensions, spirits, syrups, elixirs, including soft extracts, and the like), and examples of the solid agent include tablets, pills, powders, fine granules, granules, tablets, capsules (including hard capsules and soft capsules), troches, chewables, dry extracts, and the like. When the affective disorder ameliorating agent of the present invention is solid, it may be in a sustained or sustained release dosage form, or may be mixed with water or the like at the time of administration (ingestion).

The affective disorder ameliorating agent of the present invention can also be used as an additive or the like that adds an additional value to foods, pharmaceuticals, quasi-drugs, and the like. Examples of foods to which the affective disorder ameliorating agent of the present invention is blended include general foods and beverages and health functional foods (including foods for specified health use, nutritive functional foods, foods with function claims, and the like). Among them, the affective disorder ameliorating agent of the present invention contains flavonoids which can be ingested as a food component as an active ingredient, and therefore is preferably blended in a health functional food which can be easily ingested daily and/or continuously. In addition, the affective disorder ameliorating agent of the present invention exhibits an effect based on the action of reducing PKCγ phosphorylation (normalizing PKCγ activity), and therefore is particularly preferably blended in a food for specified health use or a food with function claims.

### Production method

The method for producing an affective disorder ameliorating agent of the present invention may be carried out according to a conventionally known normal formulation procedure according to various forms, properties, and intended use using the above active ingredients and other ingredients blended as necessary.

### Use

The affective disorder ameliorating agent of the present invention is used for the purpose of ameliorating affective disorders. Use for the purpose of ameliorating affective disorders includes the pharmaceutical use of treating affective disorders as disease and the non-pharmaceutical use of ameliorating affective disorders as presymptomatic disease. It is particularly desirable that the affective disorder is a disease state (disease) or condition (presymptomatic disease) including mania. Examples of the disease state including mania include bipolar disorder (which refers to a disease state in which a depression state and a manic state or a hypomanic state are observed), mania (which refers to a manic psychosis in which a manic state extremely appears), and hypomania (which refers to a disease state in which a manic state is milder than a manic psychosis), and examples of the presymptomatic disease including mania include a manic state in a transition stage of the disease state from a normal state. The affective disorders in the present invention preferably exclude affective disorders accompanied by dementia and affective disorders accompanied by initial symptoms of dementia (for example, mild cognitive impairment).

### Dose

The dose of the affective disorder ameliorating agent of the present invention is, for example, 0.1 g/day/60 kg or more, preferably 0.3 g/day/60 kg or more, more preferably 0.5 g/day/60 kg or more, and still more preferably 1 g/day/60 kg or more in terms of PKCγ activity inhibitor in a dose to humans.

The administration (ingestion) method of the affective disorder ameliorating agent of the present invention is not particularly limited, and for example, the administration (ingestion) can be performed once or multiple times a day orally or parenterally, and preferably once or 2 to 3 times a day orally.

### 2. PKCγ activity inhibitor

Among the isoflavones used as the PKCγ activity inhibitor as the active ingredient in the above affective disorder ameliorating agent of the present invention, the PKCγ activity inhibitory action is not known for baicalin (the above formula (2)), baicalein (the above formula (3)), luteolin (the above formula (4)), apigenin (the above formula (5)), apigenin glucoside (the above formula (6)), and salts thereof. Therefore, the present invention further also provides a PKCγ activity inhibitor containing, as an active ingredient, a compound selected from the group consisting of baicalin, baicalein, luteolin, apigenin, apigenin glucoside, and salts thereof. These PKCγ activity inhibitors can be used for amelioration of any disease state (disease) or condition (presymptomatic disease) caused by abnormal PKCγ activity. Specific examples of the disease state caused by abnormal PKCγ activity include Parkinson's disease and spinocerebellar ataxia type 14.

### Examples

Hereinafter, the present invention will be more specifically described with reference to Examples, but the present invention is not limited thereto.

### [Test Example 1]

### (Affective disorder model mice)

It has been found by the present inventor that DGKβ, which is one subtype among diacylglycerol kinase (DGK) of which 10 subtypes have been found in mammals, is abundant particularly in the hippocampus, cerebral cortex, and striatum of the brain, and rapidly increased in a period in which a neural network is developed, and also is expressed in a neuron-specific manner and promotes neurite outgrowth. Mice in which DGKβ was knocked out (DGKβKO mice) were prepared as an affective disorder (mania) model. This model mice are not associated with dementia.

### (Affective disorder evaluation system 1)

An elevated plus maze test was used. In an elevated plus maze, a passage with a wall (closed arm) and a passage without a wall (open arm) crossed each other in a cross shape, and normal mice (WT) were afraid of the open arm and stayed in the closed arm for a long time, while affective disorder model mice were less likely to feel anxiety and stayed in the open arm for a long time. Based on this, the ratio of the residence time in the open arm in the test time was used as an index of anxiety. The ratio of the residence time in the open arm significantly increased in the affective disorder model mice as compared with the normal mice, indicating mania-like behavior.

### (Affective disorder evaluation system 2)

A light/dark box test was used. The behavior of the mice was observed from the state in which the mice were placed in the dark box. Since the mice prefer a dark place, the number of times of entry into the light box is not large, but the affective disorder model mice are less likely to feel anxiety, and significant reduction in the number of times of entry into the light box and the time required to move to the light box for the first time was observed in the affective disorder model mice as compared with the normal mice.

### (Effect of lithium administration (reference test))

Male affective disorder model mice aged 8-11 weeks were allowed to freely drink water containing 600 mg/L of lithium for 10 consecutive days.

The affective disorder model mice orally administered with lithium were subjected to affective disorder evaluation system 1 (elevated plus maze test) to evaluate amelioration of affective disorder (mania). As a result, it was confirmed that the ratio of the residence time in the open arm significantly decreased in the case of taking lithium (decreased to about 30% of that in the case of taking placebo), and the affective disorder was ameliorated.

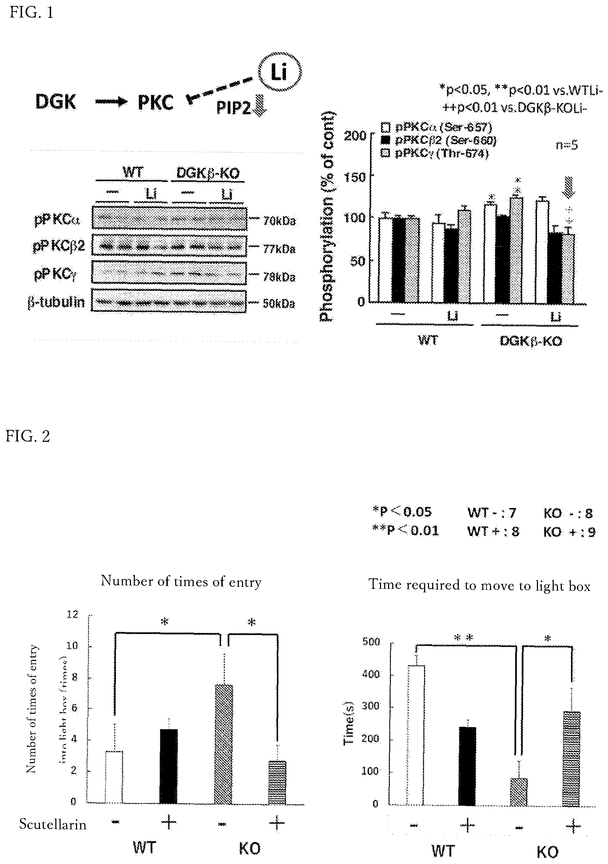

Furthermore, the cerebral cortex of mice administered with lithium was subjected to Western blotting to evaluate PKC activation. The results are shown in Fig. 1. In the figure, WT represents the results of the normal mice, DGKβ-KO represents the results of the affective disorder model mice, - represents the results without lithium administration, and Li represents the results with lithium administration. As shown in Fig. 1, it was confirmed that among PKCα and PKCγ in which phosphorylation was enhanced in the affective disorder model mice, the phosphorylation of PKCγ was reduced by the administration of lithium.

Mice orally administered with a PKCγ activity inhibitor, scutellarin, at a dose of 100 mg/mouse were subjected to affective disorder evaluation system 2 (light/dark box test) to evaluate amelioration of affective disorder (mania). The results are shown in Fig. 2. In the figure, WT represents the results of the normal mice, KO represents the results of the affective disorder model mice, - represents the results without scutellarin administration, and + represents the results with scutellarin administration. In addition, a numerical value described in a format such as "WT -: 7" represents the number of mice (the same applies to the following figures).

As shown in Fig. 2, in the affective disorder model mouse group without scutellarin administration, a significant increase in the number of times of entry into the light box and a significant reduction in the time required to move to the light box for the first time were observed, and the behavior of affective disorder was confirmed. In contrast, in the affective disorder model mouse group with scutellarin administration, significant recovery was observed in both of these items. In particular, the number of times of entry into the light box recovered to the same level as that of the normal mice without scutellarin administration, and it was recognized that the self-control of behavior due to the manic state was particularly remarkable.

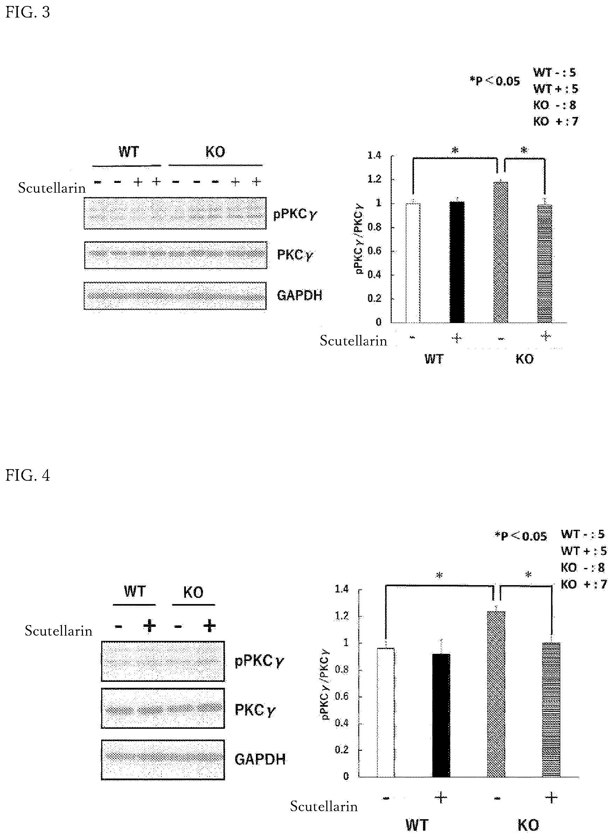

The cerebral cortex and hippocampus of mice administered with scutellarin were subjected to Western blotting, and activation of PKC was evaluated. The results are shown in Fig. 3 (cerebral cortex) and Fig. 4 (hippocampus).

As shown in Figs. 3 and 4, it was observed that abnormal activity of PKCγ in the cerebral cortex and hippocampus was observed in the affective disorder model mouse group without scutellarin administration, whereas it was recognized that the activity of PKCγ was improved to a normal level in the affective disorder model mouse group with scutellarin administration.

### [Test Example 2]

The PKCγ activity inhibitory action of various flavonoids was tested. Scutellarin (Scu), baicalin (Bai), luteolin (Lut), and apigenin (Api) were used as various flavonoids. A plasmid encoding GFP-PKCγ was introduced into COS7 cells by electroporation and cultured for 48 hours to express GFP-PKCγ. After treating the cells with TPA (12-O-Tetradecanoylphorbol-13-acetate, activator of PKCγ) and each flavonoid, the cells were ground and proteins were subjected to SDS-PAGE. Thereafter, Western blotting was performed for PKCγ and phosphorylated PKCγ (activated form).

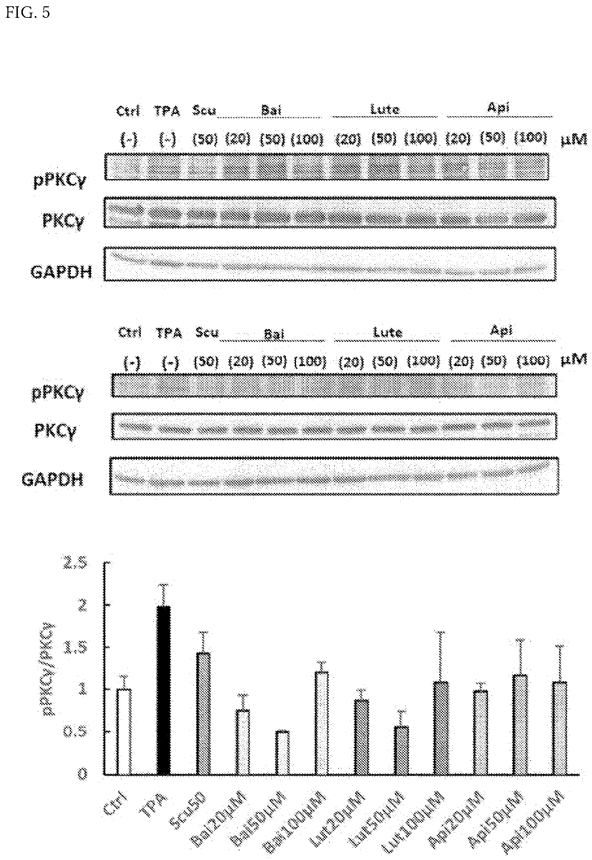

The obtained electrophoresis results (n=2) are shown in Fig. 5. As shown in Fig. 5, the PKCγ activity inhibitory action was observed not only for scutellarin (Scu) but also for baicalin (Bai), luteolin (Lut), and apigenin (Api). From this result, even when baicalin (Bai), luteolin (Lut), and apigenin (Api) are administered to the affective disorder model mice of Test Example 1, a similar affective disorder ameliorating effect can be reasonably inferred.

## Claims

1. An affective disorder ameliorating agent comprising a PKCγ activity inhibitor as an active ingredient.

2. The affective disorder ameliorating agent according to claim 1,
wherein the PKCγ activity inhibitor is selected from the group consisting of scutellarin, baicalin, baicalein, luteolin, apigenin, apigenin glucoside, and salts thereof.

3. The affective disorder ameliorating agent according to claim 1,
wherein the affective disorder is a disease state or condition including mania.

4. A food comprising the affective disorder ameliorating agent according to claim 1.

5. A PKCγ activity inhibitor comprising, as an active ingredient, a compound selected from the group consisting of baicalin, baicalein, luteolin, apigenin, apigenin glucoside, and salts thereof.
